Europäisches Patentamt

(19) European Patent Office

Office européen des brevets

(11) **EP 0 378 188 B1**

(12) **EUROPEAN PATENT SPECIFICATION**

(45) Date of publication and mention
of the grant of the patent:
**31.07.1996 Bulletin 1996/31**

(51) Int. Cl.⁶: **C07K 2/00**, C12N 9/10,
C12Q 1/48, G01N 33/574,
C12P 21/08

(21) Application number: **90100421.8**

(22) Date of filing: **10.01.1990**

(54) **Cancer-related human galactosyltransferase, its use and diagnostics**

Mit Krebs assozierte menschliche Galactosyl transferase, ihre Verwendung sowie Diagnostik

Galactosyltransférase humaine associée au cancer, son usage et moyens de diagnostic

(84) Designated Contracting States:
**GB**

(30) Priority: **11.01.1989 JP 4476/89**

(43) Date of publication of application:
**18.07.1990 Bulletin 1990/29**

(73) Proprietor: **KONICA CORPORATION**
**Tokyo 160 (JP)**

(72) Inventors:
• **Uemura, Morito**
**Hino-shi, Tokyo 191 (JP)**

• **Yoshida, Shinya**
**Hino-shi, Tokyo 191 (JP)**
• **Ueshima, Takao**
**Hino-shi, Tokyo 191 (JP)**

(74) Representative: **Türk, Gille, Hrabal, Leifert**
**Brucknerstrasse 20**
**40593 Düsseldorf (DE)**

(56) References cited:
**EP-A- 0 226 888**      **US-A- 4 205 129**

**Description**

BACKGROUND OF THE INVENTION

I. Field of the Invention

This invention relates to a novel cancer-related human galactosyltransferase. Since the cancer-related human galactosyltransferase of the present invention is mainly found in cancer patients, the galactosyltransferase of the present invention may be employed as a cancer marker. Thus, a monoclonal antibody specific to the galactosyltransferase of the present invention may be used as a diagnostic of cancer, and the galactosyltransferase of the present invention can be used as a reagent for preparing the monoclonal antibody.

II. Description of the Related Art

Galactosyltransferase (hereinafter also referred to as "GT" for short) is an enzyme which catalyzes the transfer of galactose from uridine-5'-diphosphogalactose (UDP-galactose) to non-reducing terminals of oligosaccharides of various glycoproteins or monosaccharides, which occurs in almost all the tissues in the body. Abnormal activity of GT has been recognized in various malignant tumors and the possibility of employing the GT as a cancer marker has been investigated. As a result, it was discovered that the level of GT-II which is an isozyme of GT in serum has close relationship with the existence of a cancer. The GT-II is defined as the GT enzyme activity in a band obtained in Native-PAGE (polyacrylamide gel electrophoresis), which has slower mobility than the GT-I which mainly exists in normal humans, as described in Biochem. Biophys. Res. Common, 65(2), pp.545-551, 1975. Further, MAb3872 which is a monoclonal antibody corresponding to the GT-II has been reported (Cancer Research, 48, pp.5325-5334, 1988). However, the nature of the GT-II has not yet been characterized.

SUMMARY OF THE INVENTION

Accordingly, the object of the present invention is to provide a novel galactosyltransferase which may be employed as a cancer marker and which can be assayed with high sensitivity.

The present inventors further studied the nature of the GT-II to find that a novel GT specific to cancer exists which is contained not only in the GT-II band, but also in the GT-I band and in the origins in the Native-PAGE. The cancer-related GT has the characteristics as mentioned below.

That is, the present invention provides a substantially purified cancer-related human galactosyltransferase which is obtainable from ascites of a cancer patient by $\alpha$-lactoalbumin agarose affinity chromatography and which has a molecular weight of about 50,000 determined by SDS-polyacrylamide gel electrophoresis under reduced condition, which has a reactivity with MAb4880 (FRI BP-1758) under native conditions, has an ability to self-associate, of which reactivity with MAb4880 and the degree of self-association are promoted when it is heated under reducing condition so that it is detected in a fraction of a molecular weight of not less than 200,000 in gel permeation chromatography.

Since the cancer-related galactosyltransferase (hereinafter also referred to as CRGT) of the present invention mainly exists in the tissues of patients suffering from a cancer and it scarcely occurs in the tissues of normal humans, it can be employed as a cancer marker. Further, since the CRGT of the present invention can be employed as a cancer marker, a monoclonal antibody with a specificity to the CRGT of the present invention can be used as a diagnostic of a cancer. The CRGT of the present invention may also be used as a reagent for the preparation of the CRGT-specific monoclonal antibody.

BRIEF DESCRIPTION OF THE DRAWINGS

Fig. 1 shows the results of MAb4880-sandwich assay for a solution into which a gel fraction of the band of the GT of the present invention or the normal human-originated GT was immersed, which band was obtained in Native-PAGE;

Fig. 2 shows the results of MAb4880-sandwich assay for a solution into which a gel fraction of the band of the non-treated GT of the present invention or the GT of the present invention after the treatment with 2-mercaptoethanol under heat was immersed, which band was obtained in Native-PAGE; and

Fig. 3 is an elution curve obtained in a gel permeation chromatography of the GT of the present invention after the treatment with 2-mercaptoethanol under heat.

DETAILED DESCRIPTION OF THE PREFERRED EMBODIMENTS

The CRGT of the present invention has a molecular weight of about 50,000 determined by SDS-PAGE under reducing condition after the treatment with 2-mercaptoethanol, as concretely described in the examples hereinbelow presented. This molecular weight of the CRGT of the present invention is identical with that of the normal human GT.

The CRGT of the present invention has a reactivity under the native conditions with MAb4880 which is a monoclonal antibody of which corresponding antigen is GT purified from ascites of a patient suffering from ovary cancer. The MAb4880 is prepared by the same manner as described in Cancer Research, vol. 48, p.5325, 1988, and disclosed in Japanese Laid Open Patent Application (Kokai) No. 174,100/87, which are herein incorporated by reference. A hybridoma producing the MAb4880 is deposited with Fermentation Research Institute in Japan in accordance with the Budapest Treaty under an accession number of BP-1758. The reactivity with the MAb4880 can be examined by the Western blotting method employing the MAb4880 as described in detail in the examples later described.

CRGT of the present invention has an ability to self-associate in native conditions. That is, two or more of the same protein molecules associate each other via an intermolecular interaction, so that associated molecules with a molecular weight larger than that of the non-associated single molecule by a factor of an integral number are detected. The binding between the protein molecules is not attained by a covalent bond and the associated molecules can be dissociated in certain conditions. This may be confirmed by the Western blotting as described in detail in the examples later described.

The reactivity of the CRGT of the present invention with the MAb4880 is promoted by a reduction treatment such as a treatment with 10 mM 2-mercaptoethanol. This may be confirmed by the SDS-PAGE after the reduction treatment or by the Western blotting method employing the MAb4880, which are described in detail in the examples later described.

The self-association of the CRGT of the present invention is enhanced by the above-described heat treatment under reducing condition so that the CRGT is detected in a fraction corresponding to a molecular weight of not less than 200,000 in a gel permeation chromatography, as detailed in the examples hereinbelow described.

The substrate and the function of the CRGT of the present invention are the same as those of the normal human GT. That is, the CRGT of the present invention catalyzes the reaction of the following equation:

$$\text{UDP-galactose} + \text{Acceptor} \rightarrow \text{Galactose-receptor} + \text{UDP}$$

(wherein the receptor is N-acetylglucosamine or an oligosaccharide, glycoprotein or a glycolipid having N-acetylglucosamine on a non-reducing terminals). Further, the optimal pH and the stable pH range of the CRGT are the same as those of the normal human GT, i.e., pH 6.0 - 8.0.

The CRGT of the present invention is obtainable from ascites of a cancer patient, e.g., a patient suffering from ovary cancer, by $\alpha$-lactoalbumin affinity chromatography in accordance with the method described in Cancer Research, vol. 48, p.5325, 1988 in the same manner as the normal human GT is purified, as described in detail in the examples hereinbelow described. It should be noted, however, the CRGT of the present invention exists not only in the patient suffering from ovary cancer but also in the patients suffering from other cancers such as bladder cancer, colorectal cancer, lung cancer, pancreas cancer, esophagus cancer and liver cancer. Further, the CRGT of the present invention occurs not only in ascites but also other body fluids such as serum.

The invention will now be described by way of examples thereof. It should be understood that the present invention is not limited to the examples below.

Example 1

From 1 liter of ascites from a patient suffering from ovary cancer or from 2 liters of pooled normal human serum, GT was purified by $\alpha$-lactoalbumin affinity chromatography in accordance with the description in Cancer Research, vol. 48, p.5325, 1988, to obtain 1.1 ml or 0.8 ml of GT, respectively. More particularly, the ascites or the serum was dialyzed overnight against purified water at 4°C and the insoluble mass was removed by centrifugation. To the resulting solution, Tris-buffer (pH 7.2), manganese chloride ($MnCl_2$) and N-acetylglucosamine were added to attain the final level of 20 mM, 10 mM and 5 mM, respectively, and the resulting mixture was applied to an $\alpha$-lactoalbumin affinity column (2.5 cm x 50 cm). After the column was washed with 1 liter of the same buffer as mentioned above containing manganese chloride and N-glucosamine, elution was carried out with the same buffer but not containing N-acetylglucosamine to obtain GT fractions. The same chromatography operation as described above was repeated for the obtained GT fractions to further purify the GT. The resulting GT fractions were applied to an anti-human IgG agarose column (0.5 x 3 cm) in order to remove immunoglobulins, followed by being concentrated. The obtained GT originated from the cancer patient and the normal human had a protein level of 0.8 mg/ml and 0.5 mg/ml, respectively. Ten microliters each of the obtained GT originated from the cancer patient or the normal human was treated with dodecyl sodium sulfate (SDS) and 2-mercaptoethanol and the resultants were subjected to 4 - 20% SDS-PAGE on a slab gel sizing 10 cm x 10 cm (commercially available from Daiichi Kagaku Yakuhin, Co., Ltd.) under the following conditions, followed by silver staining:

Buffer for Electrophoresis:
25 mM Tris, 192 mM glycine, 0.1% SDS (pH 8.4)
Electric Current:
60 mA constant current
Electrophoresis Time:
1 hour

As a result, both of the GT originated from the cancer patient and the GT originated from normal human exhibited a band corresponding to a molecular weight of 48,000 - 55,000, and other bands of impurities were not observed.

On the other hand, in place of the staining with silver, Western blotting was carried out in accordance with Proc. Natl. Acad. Sci. USA 76, 4350 (1979). In this Western blotting, the MAb4880 labelled with horse radish peroxidase (HRP) was used as the probe antibody. As a result, a band corresponding to the band obtained in the above-described silver-staining was observed, and the GT originated from the cancer patient was colored much more intensively than the GT originated from the normal human.

From these results, it was proved that the CRGT of the present invention can be purified by $\alpha$-lactoalbumin affinity chromatography and that it has a molecular weight of about 50,000 determined by the SDS-PAGE under reducing condition as the normal human GT.

Example 2

Ten microliters each of the GT from the cancer patient and the GT from the normal human which were purified in Example 1 was subjected to 4 - 15% Native-PAGE on a slab gel sizing 10 cm x 10 cm (commercially available from Daiichi Kagaku Yakuhin, Co., Ltd.) under the following conditions, followed by silver staining:

Buffer for Electrophoresis:
25 mM Tris, 192 mM glycine (pH 8.4)
Electric Current:
30 mA constant current
Electrophoresis Time:
2 hours

As a result, as for the GT from the cancer patient, a broad band with an Rf value of 0.3 - 0.5 and a band tailing from the origin to the position of an Rf value of about 0.3 were obtained. On the other hand, as for the GT from the normal human, although the band corresponding to the Rf value of from about 0.3 - 0.5 was observed, the band tailing from the origin to the position of an Rf value of about 0.3 was observed only in the trace amount.

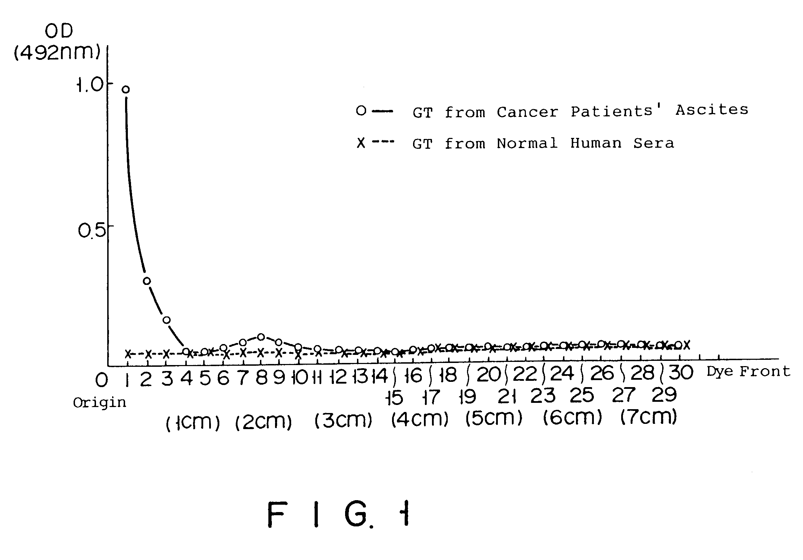

On the other hand, in place of the silver-staining, the gel (each sample lane) was cut into strips with a width of 0.25 cm, and each of the resulting strips was immersed in 100 $\mu$l of phosphate buffer (hereinafter referred to as PBS) containing 1% bovine serum albumin at 4°C overnight. Fifty microliters of each of the immersion solutions was incubated with polystyrene beads coated with MAb4880 and 200 $\mu$l of PBS at 37°C for 2 hours. The beads were then washed three times with PBS and MAb4880 labelled with horse radish peroxidase (hereinafter referred to as MAb4880-HRP) was added thereto. The mixture was allowed to react for 2 hours at room temperature. After the reaction, the beads were washed four times with PBS and o-phenylenediamine as a substrate was added to color the reaction mixture. The absorbance at 492 nm of the resulting reaction mixture was determined (MAb4880 sandwich assay). The results are shown in Fig. 1.

From these results, it was confirmed that the CRGT of the present invention has an ability to self-associate under the conditions of Native-PAGE to form associated molecules with a larger molecular weight.

Example 3

The GT from the cancer patient or from the normal human purified in Example 1 was treated with 10 mM 2-mercaptoethanol at 80°C for 3 minutes and was subjected to 4 - 15% Native-PAGE followed by silver-staining in the same manner as in Example 2. As for the GT from the cancer patient, the band tailing from the origin to the position of an Rf value of 0.3 almost disappeared and a band in the vicinity of the origin emerged in place thereof. The broad band of an Rf value of about 0.3 - 0.5 was observed as in Example 2, although the density of the silver-staining was somewhat reduced. As for the GT from the normal human, although a trace amount of a band in the vicinity of the origin was recognized, the results were not substantially changed from those in Example 2.

The gels obtained in Example 2 and the above-described gels after the heat treatment with 2-mercaptoethanol were subjected to the Western blotting as in Example 1 and were stained with MAb4880-HRP. Either of the gels obtained in Example 2 for the GT from the cancer patient or for the GT from the normal human exhibited substantially the same pattern as in the silver-staining in Example 2. In the blotting of the gels obtained in the Native-PAGE of the sample heat treated with 2-mercaptoethanol, only the band tailing from the origin was stained for the GT from the cancer patient. On the other hand, as to the GT from the normal human, only the origin was slightly stained.

From these results, it was confirmed that the self-association of the CRGT of the present invention is enhanced by the heat treatment with 2-mercaptoethanol so that its molecular weight is increased and the binding reactivity with the MAb4880 is prominently enhanced by the heat treatment with 2-mercaptoethanol and that the binding reactivity of the GT from normal human with the MAb4880 is reduced by the heat treatment with 2-mercaptoethanol.

Example 4

The GT from the cancer patient or the GT from the normal human purified in Example 1 was treated with 10 mM 2-mercaptoethanol at 80°C for 3 minutes. The GT was 10-fold to about 7000-fold diluted with 1% BSA-PBS and was subjected to the sandwich assay using the MAb4880 as in Example 2. In another run, the GT was subjected to the sandwich assay without the heat treatment with 2-mercaptoethanol.

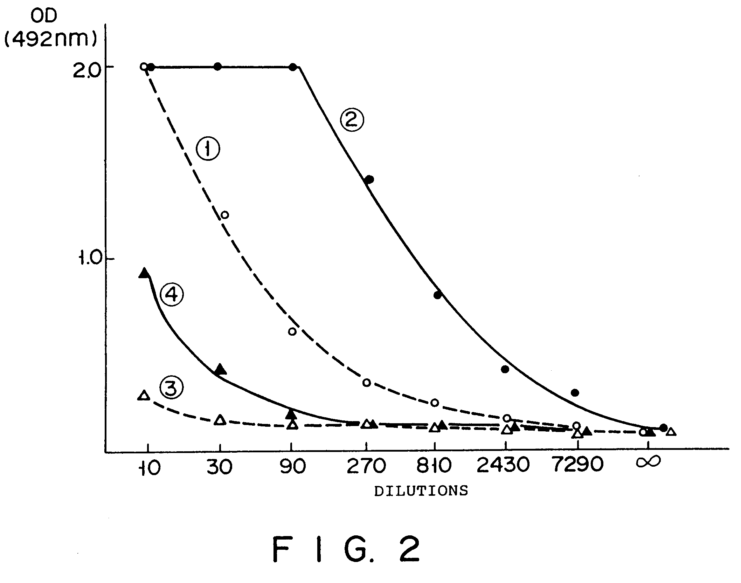

The results are shown in Fig. 2. In Fig. 2, curve 1 shows the results for the GT from the cancer patient without the treatment, curve 2 shows the results for the GT from the cancer patient which was subjected to the heat treatment with 2-mercaptoethanol, curve 3 shows the results for the GT from the normal human without the treatment and curve 4 shows the results for the GT from the normal human which was subjected to the heat treatment with 2-mercaptoethanol.

From these results, it was confirmed that the detection sensitivity in the MAb4880 sandwich assay of the CRGT of the present invention was drastically promoted by the 2-mercaptoethanol heat treatment, and that a large amount of the CRGT of the present invention exists in the ascites of cancer patients, although a small amount of it also exists in the serum of the normal humans.

Example 5

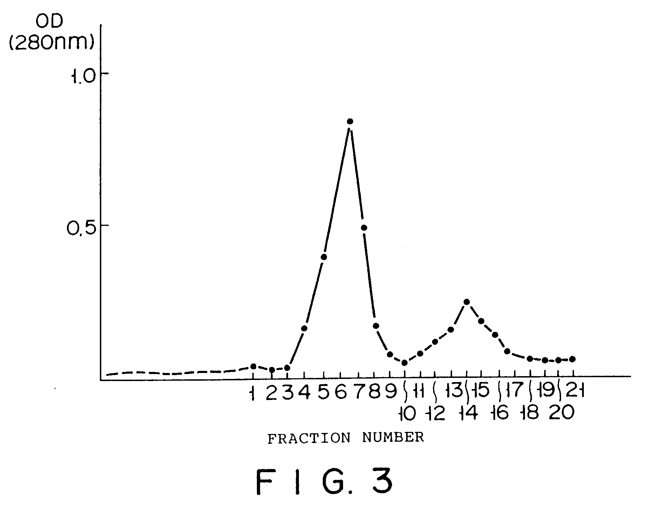

One hundred microliters aliquotes of each of the samples for the sandwich assay obtained in Example 4 were subjected to gel permeation chromatography on FPLC super rose-12 (commercially available from Pharmacia) using PBS as the solvent at a flow rate of 0.5 ml/min. The elution curve of the GT from the cancer patient heat treated with 2-mercaptoethanol is shown in Fig. 3. Two microliters aliquotes of the 20 fractions obtained from 10 minutes to minutes from the commencement of the elution were directly blotted to nitrocellulose membranes and were reacted with MAb-4880 to color the same. The results are shown in Table 1. The intensity of the reaction is expressed by the following symbols.

++ :    Strongly Colored
+ :     Weakly Colored
± :     Slightly Colored
- :     Not Colored

As is apparent from Table 1, the self-association of the CRGT of the present invention is enhanced by the 2-mercaptoethanol treatment and so its molecular weight is increased so that it is detected in the fractions corresponding to a molecular weight of not less than 200,000 in the gel permeation chromatography.

TABLE 1

| | Molecular Weight | | | | ∞ void | 1M. | | 500 T. | | | 200 T. | | | 100 T. | | | 50 T. | | | |
|---|---|---|---|---|---|---|---|---|---|---|---|---|---|---|---|---|---|---|---|---|
| | Fraction No. | 1 | 2 | 3 | 4 | 5 | 6 | 7 | 8 | 9 | 10 | 11 | 12 | 13 | 14 | 15 | 16 | 17 | 18 | 19 | 20 |
| GT from Cancer | 2-ME and heat non-treated | - | - | - | - | ± | + | ± | - | - | - | - | - | - | - | ± | ± | - | - | - | - |
| Patient | 2-ME and heat treated | - | - | - | ± | + | ++ | + | ± | - | - | - | - | - | - | - | - | - | - | - | - |
| GT from Normal | 2-ME and heat non-treated | - | - | - | - | - | - | - | - | - | - | - | - | - | - | - | ± | ± | - | - | - |
| Human | 2-ME and heat treated | - | - | - | - | - | ± | - | - | - | - | - | - | - | - | - | - | - | - | - | - |

2-ME: 2-mercaptoethanol

M.: Million

T.: Thousand

EP 0 378 188 B1

Example 6

The monoclonal antibody MAb3872 (ATCC HB 8945) specific to the GT-II described in Cancer Research vol. 48, p.5325, 1988 was immobilized in an agarose gel activated with cyanogen bromide to prepare an affinity chromatography column (2.5 cm x 10 cm). About one liter of ascites of a patient suffering from ovary cancer was passed through the column at a flow rate of 50 ml/hr and the column was then washed with 500 ml of phosphate buffer (pH 7.3) containing 0.5 M NaCl. Then the elution was carried out with a glycine buffer (pH 2.8) containing 0.5M NaCl and the fractions exhibiting the reactivity with MAb3872 were pooled, followed by being concentrated to a volume of about 1 ml.

The sample purified by the MAb3872 affinity chromatography and the sample purified by the affinity chromatography in Example 1 were subjected to the assay mentioned below. The MAb4880-immobilized beads and the MAb4880-HRP were those prepared in Example 1. The MAb3872-immobilized beads and the MAb3872-HRP labelled substance were prepared in the same manner as in the case of MAb4880. The sample purified by the $\alpha$-lactoalbumin was 500-fold diluted with 1% BSA-PBS and the sample purified by MAb-3872 affinity column was 100-fold diluted with the same buffer. The sandwich assay was conducted as in Example 2 for the combination of the immobilized beads and the labelled substance shown in Table 2. The determined absorbance is shown in Table 2.

As is apparent from Table 2, the CRGT of the present invention is different from GT-II with a reactivity with MAb3872, although some of the characteristics are common.

TABLE 2

| Immobilized Beads | MAb3872 | MAb3872 | MAb4880 |
|---|---|---|---|
| Labelled Substance | MAb3872-HRP | MAb4880-HRP | MAb4880-HRP |
| Purified Product A | 0.082 | 0.904 | 1.890 |
| Purified Product B | 1.545 | 0.281 | 0.045 |
| Purified Product A: GT purified by $\alpha$-lactoalbumin affinity chromatography Purified Product B: GT purified by MAb3872 affinity chromatography | | | |

**Claims**

1. A substantially purified cancer-related human galactosyltransferase which is obtainable from ascites of a cancer patient by $\alpha$-lactoalbumin agarose affinity chromatography and which has a molecular weight of about 50 000 determined by SDS-polyacrylamide gel electrophoresis under reduced condition, which has a reactivity with MAb4880 (FRI BP-1758) under native conditions, has an ability to self-associate, of which reactivity with MAb4880 and the degree of self-association are promoted when it is heated under reducing conditions so that it is detected in a fraction of a molecular weight of not less than 200 000 in gel permeation chromatography.

2. Use of the cancer-related human galactosyltransferase of claim 1 as a cancer marker.

3. Use of the cancer-related human galactosyltransferase of claim 1 for preparing monoclonal antibodies.

4. Use of the cancer-related human galactosyltransferase of claim 1 for the preparation of diagnostics of cancer.

5. Diagnostic, containing the cancer-related human galactosyltransferase of claim 1 or antibodies prepared by its use.

**Patentansprüche**

1. Eine im wesentlichen gereinigte krebsbedingte Human-Galactosyltransferase, die aus Ascites eines Krebspatienten durch $\alpha$-Lactoalbumin-Agarose-Affinitätschromatographie erhältlich ist, und die ein Molekulargewicht von etwa 50 000, bestimmt durch SDS-Polyacrylamid-Gelelektrophorese unter reduzierenden Bedingungen, eine Reaktivität mit MAb4880 (FRI BP-1758) unter nativen Bedingungen, und Fähigkeit zur Selbstassoziierung aufweist, und deren Reaktivität mit MAb4880 und deren Grad der Selbstassoziierung durch Erhitzen unter reduzierenden Bedingungen gefördert wird, so daß sie in einer Fraktion des Molekulargewichts von nicht weniger als 200 000 bei der Gelpermeationschromatographie nachgewiesen wird.

2. Verwendung der krebsbedingten Human-Galactosyltransferase aus Anspruch 1 als Krebsmarker.

3. Verwendung der krebsbedingten Human-Galactosyltransferase aus Anspruch 1 für die Herstellung monoclonaler Antikörper.

4. Verwendung der krebsbedingten Human-Galactosyltransferase aus Anspruch 1 für die Herstellung von Diagnostika für Krebs.

5. Diagnostikum, enthaltend die krebsbedingte Human-Galactosyltransferase aus Anspruch 1 oder durch ihre Verwendung hergestellte Antikörper.

**Revendications**

1. Galactosyl-transférase humaine liée au cancer, sensiblement purifiée, que l'on peut obtenir à partir de l'ascite d'un malade atteint de cancer par une chromatographie d'affinité sur agarose d'$\alpha$-lactoalbumine et qui a un poids moléculaire d'environ 50 000 déterminé par électrophorèse sur gel de SDS-polyacrylamide dans des conditions réduites, qui a une réactivité avec MAb4880 (FRI BP-1758) dans les conditions natives, a la capacité de s'auto-associer, dont la réactivité avec MAb4880 et le degré d'auto-association sont favorisés lorsqu'on la chauffe dans des conditions réductrices afin qu'on la détecte dans une fraction d'un poids moléculaire d'au moins 200 000 dans une chromatographie de perméation des gels.

2. Application de la galactosyl-transférase humaine liée au cancer de la revendication 1 comme marqueur du cancer.

3. Application de la galactosyl-transférase humaine liée au cancer de la revendication 1 à la préparation d'anticorps monoclonaux.

4. Application de la galactosyl-transférase humaine liée au cancer de la revendication 1 à la préparation de trousses de diagnostic du cancer.

5. Trousse de diagnostic contenant la galactosyl-transférase humaine liée au cancer de la revendication 1 ou des anticorps préparés en l'utilisant.

F I G. 1

EP 0 378 188 B1

F I G. 2

EP 0 378 188 B1

F I G. 3